# FASCICULE DE BREVET EUROPEEN

(11) **EP 3 487 476 B1**
(45) Date de publication et mention de la délivrance du brevet: **04.08.2021**
(21) Numéro de dépôt: 17735584.9
(22) Date de dépôt: 10.07.2017
(51) Int. Cl.: A61K 8/73, A61Q 19/00, A61K 8/11, A23P 10/30

(54) **SÉRIE DE CAPSULES ET PROCÉDÉ DE FABRICATION, COMPOSITION COSMÉTIQUE ET TRAITEMENT COSMÉTIQUE**
SERIE VON KAPSELN UND VERFAHREN ZUR HERSTELLUNG, KOSMETISCHE ZUSAMMENSETZUNG UND KOSMETISCHE BEHANDLUNG
SERIES OF CAPSULES AND METHOD OF MANUFACTURE, COSMETIC COMPOSITION AND COSMETIC TREATMENT

(30) Priorité: 20.07.2016 FR 1656911
(43) Date de publication de la demande: 29.05.2019
(73) Titulaire: Capsum, 13013 Marseille (FR)
(72) Inventeur: PAFUMI, Yan Eric, 13120 Gardanne (FR); SANTANACH CARRERAS, Enric, 65290 Louey (FR)
(74) Mandataire: Lavoix
(86) Numéro de dépôt international: PCT/EP2017/067276
(87) Numéro de publication internationale: WO 2018/015197

(56) Documents cités:
- WO-A1-2012/089820
- WO-A1-2015/075074
- FR-A1- 3 024 658

## Description

La présente invention concerne le domaine des compositions, notamment cosmétique, et plus précisément le domaine des procédés de fabrication associés ainsi que les procédés d'application sur une matière kératinique desdites compositions.

La présente invention concerne plus particulièrement un procédé de fabrication d'une série de capsules, chaque capsule comprenant un cœur liquide ou au moins en partie gélifié ou au moins en partie thixotrope, et une enveloppe gélifiée encapsulant totalement le cœur.

L'invention concerne également une série de capsules susceptible d'être fabriquée par un tel procédé, et une composition, notamment cosmétique, comprenant une telle série de capsules et un milieu de réception de ces capsules.

L'invention concerne enfin un procédé non thérapeutique de traitement cosmétique d'une matière kératinique, en particulier de la peau, comprenant au moins une étape d'application sur ladite matière kératinique d'au moins cette composition.

La demanderesse fabrique et commercialise déjà des compositions cosmétiques sous forme d'émulsion reposant sur la mise en œuvre de capsules, ou perles, obtenues par des procédés millifluidiques.

Selon une première technologie, les capsules présentent une enveloppe extérieure d'alginate de calcium et un cœur liquide ou gélifié. Un tel procédé est avantageux en ce que les capsules, d'un diamètre compris entre 1 et 3 mm, ont une monodispersité apparente et une enveloppe présentant une faible épaisseur. Cette technologie, incluant un procédé de fabrication associé, est décrite dans les documents WO 2010/063937, WO 2011/086331 et WO 2015/071433.

Le procédé présente toutefois quelques limitations, notamment au niveau de la texture et de la nature du liquide à encapsuler. Il ne permet d'encapsuler qu'un seul type d'actif à la fois, à savoir aqueux ou huileux.

Ainsi, la demanderesse a développé une deuxième et une troisième technologies, qui permettent de surmonter les inconvénients précités.

Dans la deuxième technologie, les capsules comprennent un cœur formé d'une goutte d'huile suspendue dans une phase aqueuse d'eau, et une fine membrane d'alginate entourant le cœur. La capsule est composée de trois phases : une phase interne huileuse, une phase intermédiaire aqueuse et une phase externe.

Dans la troisième technologie, le cœur est formé d'une goutte aqueuse suspendue dans une phase huileuse. La capsule est alors composée de trois phases : une phase interne aqueuse, une phase intermédiaire huileuse et une phase externe.

Dans les deux cas, la phase intermédiaire présente une viscosité importante, avantageusement est gélifiée, pour assurer une bonne suspension de la goutte interne dans la phase intermédiaire et éviter le contact de la phase interne avec la phase externe.

Ces deuxième et troisième technologies utilisent le principe de la co-extrusion à trois voies.

Trois fluides arrivent par trois voies différentes d'une buse et forment une goutte triphasique. Cette goutte tombe ensuite dans un bain de calcium auquel est ajouté un tensioactif, en particulier un tensioactif non-ionique, permettant une meilleure pénétration de la capsule dans le bain de calcium. La chute dans le bain entraîne la gélification de la capsule par diffusion du calcium dans la phase externe qui devient la membrane gélifiée. Les deuxième et troisième technologies ci-dessus, incluant les procédés de fabrication associés, sont notamment décrites dans les documents WO 2012/089820 et WO2013/132083.

La mise en œuvre de particules, notamment les capsules décrites ci-dessus, dans des compositions, notamment cosmétiques, est connue et confère à ces dernières un aspect visuel différenciant et particulièrement attractif, tout en protégeant les actifs encapsulés.

Le caractère différenciant et attractif est parfois encore renforcé par la mise en œuvre, dans la membrane et/ou le cœur liquide desdites capsules, d'agents colorants tels que des pigments, des nacres, des colorants, des matériaux à effet optique, en particulier des cristaux liquides, et leurs mélanges.

Néanmoins, le développement et la mise à disposition des consommateurs de compositions présentant un aspect visuel inédit demeurent un objectif constant.

Un but de l'invention est donc de fournir des particules dotées d'une structure nouvelle leur conférant un aspect visuel inédit et un procédé de fabrication de ces particules, la fabrication devant être d'un coût compétitif.

A cet effet, l'invention a pour objet un procédé selon la revendication 1.

Selon des modes de réalisation particuliers, le procédé comprend l'une ou plusieurs des caractéristiques correspondant aux revendications 2 à 9, prise(s) selon toutes les combinaisons techniquement possibles.

L'invention a également pour objet une série de capsules selon la revendication 10.

Selon des modes de réalisation particuliers, la série de capsules comprend l'une ou plusieurs des caractéristiques correspondant aux revendications 11 à 13, prise(s) selon toutes les combinaisons techniquement possibles.

L'invention a également pour objet une composition, notamment cosmétique, comprenant une série de capsules telle que définie précédemment, et un milieu de réception de ces capsules.

L'invention a aussi pour objet un procédé selon la revendication 15.

L'invention sera mieux comprise à la lecture de la description qui va suivre, donnée uniquement à titre d'exemple et faite en se référant aux dessins annexés, sur lesquels :
- la figure 1 est une vue schématique en perspective d'une capsule d'une série selon un premier mode de réalisation de l'invention,
- la figure 2 est une vue schématique en coupe sensiblement selon un plan sensiblement équatorial de la capsule représentée sur la figure 1,
- la figure 3 est une vue schématique en perspective d'une capsule d'une série selon un deuxième mode de réalisation de l'invention,
- la figure 4 est une vue schématique en perspective d'une capsule d'une série selon un troisième mode de réalisation de l'invention,
- la figure 5 est une vue schématique en coupe sensiblement selon un plan équatorial de la capsule représentée sur la figure 4,
- la figure 6 est une vue schématique en perspective d'une capsule d'une série selon une variante du troisième mode de réalisation,
- la figure 7 est une vue schématique d'un dispositif mettant en œuvre un procédé de fabrication selon l'invention permettant de fabriquer la capsule représentée sur les figures 1 et 2,
- la figure 8 est une vue schématique d'une variante du dispositif représenté sur la figure 7, permettant de fabriquer la capsule représentée sur les figures 4 et 5,
- la figure 9 est une vue schématique du dispositif représenté sur la figure 8 et utilisé pour fabriquer la capsule représentée sur la figure 6,
- les figures 10 et 11 sont des vues schématiques d'autres variantes du dispositif représenté sur la figure 7, et
- la figure 12 est une vue schématique d'une variante du dispositif représenté sur la figure 9.

### Premier mode de réalisation des capsules

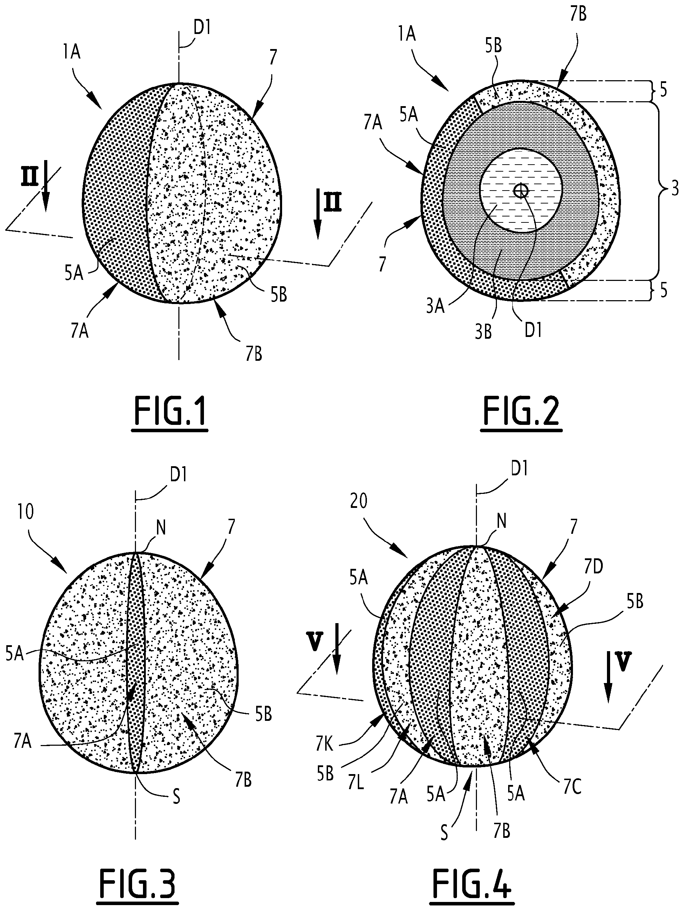

En référence aux figures 1, 2 et 7, on décrit une série de capsules 1A, 1B, 1C selon un premier mode de réalisation de l'invention.

Les capsules 1A, 1B, 1C étant analogues les unes aux autres, seules la capsule 1A représentée sur les figures 1 et 2 sera décrite ci-après.

Selon un mode de réalisation particulier (non représenté), les capsules d'une série ne sont pas analogues entre elles. Une telle série est par exemple obtenu en mélangeant des capsules d'une ou plusieurs séries telles que décrites dans le présent document.

De préférence, la capsule 1A présente une forme sensiblement sphérique et un diamètre extérieur supérieur à 0,5 mm, avantageusement inférieur à 10 mm et préférentiellement compris entre 1 mm et 5 mm.

La capsule 1A comprend un cœur 3 liquide ou au moins en partie gélifié ou au moins en partie thixotrope, et une enveloppe gélifiée 5 encapsulant totalement le cœur.

Avantageusement, le rapport volumique Rᵥ du volume du cœur 3 au volume de l'enveloppe gélifiée 5 est supérieur à 2, et est notamment supérieur à 5. Ce rapport Rᵥ est avantageusement inférieur à 50. Il est par exemple compris entre 5 et 10.

Dans cet exemple, le cœur 3 comporte deux phases internes 3A, 3B.

La phase interne 3B entoure complètement la phase interne 3A, et constitue une phase intermédiaire entre la phase interne 3A et l'enveloppe gélifiée 5.

L'enveloppe gélifiée 5 définit une surface externe 7, les deux phases externes 5A, 5B formant deux portions 7A, 7B distinctes de la surface externe.

Chaque phase externe 5A, 5B comprend au moins un polyélectrolyte gélifié, voire contient un polyélectrolyte gélifié, identique ou différent l'un de l'autre.

Au moins l'une des deux phases externes 5A, 5B, par exemple la phase externe 5A, comporte en outre au moins un agent colorant.

D'une manière plus générale, les phases externes 5A, 5B diffèrent l'une de l'autre par la présence ou l'absence et/ou la nature et/ou la teneur en agent(s) colorant(s).

Les phases externes 5A, 5B sont par exemple en contact avec le cœur 3.

Selon une variante non représentée, certaines des phases externes, par exemple la phase externe 5B, ne sont pas en contact avec le cœur 3.

L'agent colorant permet de distinguer à l'œil la portion 7A de la portion 7B et donc de conférer aux capsules selon l'invention un aspect visuel inédit.

L'agent colorant est par exemple choisi parmi les pigments, les nacres (paillettes), les matériaux à effet optique, en particulier les cristaux liquides, et leurs mélanges, de préférence les pigments et/ou les nacres. Par exemple, la portion 7A est colorée par l'agent colorant, alors que la portion 7B ne l'est pas.

Selon une variante non représentée, les deux portions 7A, 7B sont toutes les deux colorées, mais de couleurs différentes, respectivement par un agent colorant ou un mélange d'agents colorants.

Dans cet exemple, les portions 7A, 7B présentent des aires sensiblement égales (par exemple à +/- 5%). Les portions 7A, 7B sont par exemple sensiblement hémisphériques.

### Cœur

Le cœur 3 de la capsule 1A comprend un ou plusieurs agents actifs, en mélange ou dans des phases séparées, ou en plusieurs mélanges dans des phases séparées.

Dans le cadre de la présente description, on entend par « agent actif » un composé ayant un effet physiologique bénéfique sur l'élément sur lequel il agit. Il vise par exemple à protéger, maintenir en bon état, soigner, guérir, parfumer ou aromatiser.

L'agent actif est avantageusement un agent cosmétique, dermo-pharmaceutique, pharmaceutique, parfumant ou alimentaire.

Le cœur 3 peut contenir l'agent actif sous forme de liquide pur, ou une solution de l'agent actif dans un solvant liquide, ou une dispersion telle qu'une émulsion ou une suspension de l'agent actif dans un liquide.

Lorsque l'agent actif est un agent cosmétique, il peut être choisi parmi le hyaluronate de sodium ou d'autres molécules hydratantes/réparatrices, des vitamines, des enzymes, des actifs anti-rides, anti-âge, protecteurs/antiradicalaires, antioxydants, apaisants, adoucissants, anti irritants, tenseurs/lissants, émollients, amincissants, anti capitons, raffermissants, gainants, drainants, anti-inflammatoires, dépigmentants, blanchissants, autobronzants, exfoliants, stimulant le renouvellement cellulaire ou stimulant la microcirculation cutanée, absorbant ou filtrant les UV, antipelliculaires, et leurs mélanges.

Un agent cosmétique pouvant être contenu dans le cœur 3 est par exemple cité dans la Directive 93/35/CEE du Conseil datée du 14 juin 1993. Ce produit est par exemple une crème, une émulsion, une lotion, un gel et une huile pour la peau (mains, visage, pieds, etc.), un fond de teint (liquide, pâte) une préparation pour bains et douches (sels, mousses, huiles, gels, etc.), un produit de soins capillaires (teintures capillaires et décolorants), un produit de nettoyage (lotions, poudres, shampoings), un produit d'entretien pour la chevelure (lotions, crèmes, huiles), un produit de coiffage (lotions, laques, brillantines), un produit pour le rasage (savons, mousses, lotions, etc.), un produit destiné à être appliqué sur les lèvres, un produit solaire, un produit de bronzage sans soleil, un produit permettant de blanchir la peau, un produit antirides.

Les agents dermo-pharmaceutiques désignent plus particulièrement les agents agissant au niveau de la peau.

Lorsque l'agent actif est un agent pharmaceutique, il est choisi avantageusement parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques, les antibiotiques et leurs mélanges.

Lorsque l'agent actif est un agent parfumant, il peut être sous la forme d'un mélange. Parmi les agents parfumants, on peut notamment citer tout type de parfum ou de fragrance, ces termes étant utilisés ici de façon indifférente. Ces parfums ou fragrances sont bien connus de l'homme du métier et incluent notamment ceux mentionnés, par exemple, dans S. Arctander, Perfume and Flavor Chemicals (Montclair, N.J., 1969), S. Arctander, Perfume and Flavor Materials of Natural Origin (Elizabeth, N.J., 1960) et dans "Flavor and Fragrance Materials", 1991 (Allured Publishing Co. Wheaton, III. USA). Les parfums utilisés dans le cadre de la présente invention peuvent comprendre les produits naturels comme les extraits, les huiles essentielles, les absolus, les résinoïdes, les résines, les concrètes, etc... ainsi que les substances basiques de synthèse comme les hydrocarbures, les alcools, les aldéhydes, les cétones, les éthers, les acides, les esters, les acétals, les cétals, les nitriles, etc..., y compris les composés, saturés et insaturés, les composés aliphatiques, alicycliques et hétérocycliques.

Les agents alimentaires sont avantageusement des purées de légumes ou de fruits telles que la purée de mangue, de la purée de poire, de la purée de coco, de la crème d'oignons, de poireaux, de carottes, ou d'autres préparations pouvant mélanger plusieurs fruits ou légumes. En variante, il s'agit d'huiles telles qu'une huile alimentaire, du type huile d'olive, huile de soja, huile de grains de raisin, huile de tournesol, ou toute autre huile extraite des végétaux, ainsi que des actifs alimentaires tels que des probiotiques, des levures, des vitamines, des minéraux ou des oléoactifs.

Le cœur 3 peut également comprendre un agent colorant.

Selon un mode de réalisation, le cœur 3 est liquide.

Selon un mode de réalisation particulier, le cœur 3 est au moins en partie gélifié. Dans ce cas, le cœur est par exemple formé par la gélification d'un produit gélifiant obtenu par un changement de température, notamment par une diminution de température d'au moins 10°C. En variante, la gélification est obtenue en présence d'ions, d'autres molécules ou de certaines conditions de pH ou de force ionique.

Le cœur 3 contient par exemple un gel de polyélectrolyte identique au gel de polyélectrolyte de l'enveloppe gélifiée 5. Selon une variante, le cœur 3 contient une solution aqueuse d'un gélifiant différent du polyélectrolyte de l'enveloppe gélifiée 5. Le gélifiant est de préférence choisi dans le groupe constitué des polyosides, des galactomannanes, des polysaccharides, des glycosaminoglycanes, des polyols et leurs mélanges. Avantageusement, il est choisi dans le groupe constitué de la gomme xanthane, du carraghénane, de la caroube, de la gomme guar, de la gellane, de l'acide hyaluronique, du glycérol, du propanediol, de la cellulose ou de ses dérivés, et de leurs mélanges.

Lorsqu'un gélifiant est présent dans le cœur 3, il est typiquement présent selon une concentration comprise de 0,01% à 1% en masse par rapport à la masse totale du cœur.

Selon un autre mode de réalisation particulier, le cœur 3 est au moins en partie thixotrope. Par phase thixotrope, on entend au sens de l'invention une phase qui est à l'état liquide et déstructuré lorsqu'elle s'écoule, mais qui est sensiblement solide ou gélifié au repos.

Par « liquide lorsqu'elle s'écoule », on entend que le comportement de la phase considérée est visqueux, c'est à dire que la déformation du matériau dépend non seulement de la contrainte appliquée mais également de la durée pendant laquelle cette contrainte est appliquée. Une façon de caractériser ce comportement est par un test de fluage à l'aide d'un rhéomètre sur l'échantillon, on applique une contrainte caractéristique des écoulements mis en jeu pendant la fabrication et on trace la courbe de déformation en fonction du temps (données obtenues avec le logiciel du rhéomètre). Si la courbe a une pente non nulle aux temps longs (plus de 30 secondes), la phase peut être considérée comme étant liquide. Si cette pente est nulle, la phase peut être considérée comme étant solide.

Par « solide ou gélifié au repos », on entend que la déformation du matériau dépend seulement de la contrainte appliquée. Une façon de caractériser ce comportement est par un test de fluage à l'aide d'un rhéomètre, sur l'échantillon, on applique une contrainte caractéristique de celles subies par la capsule au repos en fonction du temps (données obtenues avec le logiciel du rhéomètre). Si la courbe a une pente nulle aux temps longs (plus de 30 secondes), la phase peut être considérée comme étant solide. Si cette pente est non nulle, la phase peut être considérée comme étant liquide.

A titre d'agent thixotrope, on peut par exemple citer les particules colloïdales (silice, argiles, latex...).

Lorsqu'un agent thixotrope est présent dans le cœur 3, il est typiquement présent selon une concentration comprise de 0,5% à 5% en masse par rapport à la masse totale du cœur, voire à la masse de la phase le comprenant.

La capsule 1A est dite « complexe », car le cœur 3 comporte au moins deux phases internes. Une capsule complexe est par exemple une capsule telle que décrite dans les demandes WO 2012/089820 et WO 2013/132083 au nom de la Demanderesse.

Selon une variante non représentée, le cœur 3 comprend une phase interne 3B continue, au sein de laquelle se trouve une pluralité de gouttes de phase(s) interne(s) 3A.

L'agent actif du cœur 3, lorsqu'il est présent, peut être contenu dans la phase interne 3A et/ou dans la phase interne 3B.

Par exemple, la phase interne 3B est aqueuse et la phase interne 3A est huileuse et non miscible avec la phase interne 3B à température ambiante et pression atmosphérique. On peut ainsi parler de cœur « huile-dans-eau ».

En variante, la phase interne 3B est huileuse et la phase interne 3A est aqueuse et non miscible avec la phase interne 3B à température ambiante et pression atmosphérique. On peut ainsi parler de cœur « eau-dans-huile ».

On entend par « phase aqueuse » une phase ayant la propriété de solubiliser des composés polaires et hydrophiles. Une phase aqueuse comprend de préférence de l'eau et au moins un agent actif tel que décrit ci-dessus, qui est par ailleurs hydrophile.

On entend par « phase huileuse » une phase ayant la propriété de solubiliser des composés apolaires, tels que des corps gras, des huiles, des lipides. Une phase huileuse comprend de préférence un corps gras, une huile ou un mélange d'huiles d'origine végétale, animale ou minérale.

A titre d'huile végétale, on peut par exemple citer l'huile d'abricot, l'huile d'amande douce, l'huile de jojoba, l'huile de palme, l'huile d'argan ou le phytosqualane.

A titre de corps gras, on peut par exemple citer les esters d'alcools gras et/ou d'acides gras, tels que le myristate d'isopropyle, le myristate de glycérol, l'isononanoate d'isononyle, les triglycérides d'acide caprylique ou d'acide caprique, le palmitate d'isopropyle et le palmitate d'éthyle.

A titre d'huile animale, on peut par exemple citer le squalène.

A titre d'huile minérale, on peut par exemple citer le polyisobutylène hydrogéné, l'isododécane, les huiles de paraffine ou les huiles de silicone.

La phase interne 3B est avantageusement liquide.

Dans une variante, la phase interne 3A est liquide et la phase interne 3B est thixotrope.

Alternativement, la phase interne 3A est liquide et la phase interne 3B est gélifiée.

La thixotropie ou la gélification de la phase interne 3B est avantageuse en ce qu'elle permet d'assurer la suspension de la goutte de phase interne 3A dans la phase intermédiaire formée par la phase interne 3B dans une capsule complexe selon l'invention. De fait, elle contribue à préserver l'intégrité de la capsule dans le temps.

La phase interne 3B peut en outre comprendre un ou plusieurs agents actifs cosmétiques, dermo-pharmaceutiques, pharmaceutiques, parfumants ou alimentaires, tels que définis plus haut.

La phase interne 3B peut également comprendre des excipients différents des gélifiants et des agents thixotropes décrits précédemment, tels que des épaississants, ou des modificateurs de rhéologie. Ces épaississants sont par exemple des polymères, des cross-polymères, des microgels, des gommes ou des protéines, dont des polysaccharides, des celluloses, des polyosides, des polymères et co-polymères à base de silicone, des particules colloïdales (silice, argiles, latex...).

La phase interne 3B peut comprendre des particules solides, et notamment des particules de nacre.

Avantageusement, la phase interne 3B est totalement interposée entre la phase interne 3A et l'enveloppe gélifiée 5. La phase interne 3B maintient la phase interne 3A totalement à l'écart de l'enveloppe gélifiée 5.

La phase interne 3A peut comprendre un ou plusieurs agents actifs cosmétiques, dermo-pharmaceutiques, pharmaceutiques, parfumants ou alimentaires, tels que définis plus haut.

La phase interne 3A présente avantageusement une forme sphérique.

En variante, phase interne 3A possède une forme elliptique ou lenticulaire.

Selon une autre variante, la capsule 1A est une capsule dite « pleine », c'est-à-dire que le cœur comprend également un polyélectrolyte à l'état gélifié chélaté par des cations divalents, ledit polyélectrolyte étant identique à celui présent dans l'enveloppe.

Typiquement, à l'exception de la nature et/ou de la teneur en agent colorant, la composition du cœur et la composition de l'enveloppe sont alors identiques et constituent une seule et même phase.

Selon une variante non représentée, la capsule 1A est une capsule dite « simple », signifiant que le cœur 3 est constitué d'une unique phase interne, aqueuse ou huileuse, au contact de l'enveloppe gélifiée 5. La phase interne est par exemple l'une des phases internes 3A, 3B décrites plus haut

Une capsule simple est par exemple décrite dans les demandes internationales WO 2010/063937, WO2011/086331 et WO 2015/071433 au nom de la Demanderesse.

### Enveloppe

Chaque phase externe 5A, 5B de l'enveloppe 5 gélifiée de la capsule 1A est une membrane gélifiée comprenant au moins un polyélectrolyte à l'état gélifié chélaté par des cations divalents qui assure la tenue mécanique de la capsule 1A.

L'enveloppe gélifiée 5 a par exemple une épaisseur inférieure à 500 µm, avantageusement supérieure à 10 µm, typiquement comprise entre 25 µm et 100 µm.

Chacune des phases externes 5A, 5B est par exemple constituée d'un hydrogel comprenant de l'eau et au moins un polyélectrolyte chélaté par des cations divalents, et éventuellement au moins un tensioactif.

Dans le cadre de la présente invention, le ou les polyélectrolytes présents dans l'enveloppe gélifiée 5 sont réactifs aux cations divalents.

Par « polyélectrolyte réactif aux cations divalents », on entend, au sens de la présente invention, un polyélectrolyte susceptible de passer d'un état liquide dans une solution aqueuse à un état gélifié sous l'effet d'un contact avec une solution gélifiante contenant des cations divalents.

Par « cations divalents », on entend notamment les cations des métaux alcalino-terreux choisis par exemple parmi les cations calcium (Ca²⁺), baryum (Ba²⁺), et magnésium (Mg²⁺). De préférence, les cations divalents sont des cations calcium (Ca²⁺).

Le polyélectrolyte peut être notamment un polysaccharide naturel réactif aux ions multivalents tel qu'un alginate d'alcalin, une gellane, une pectine ou un carraghénane.

De préférence, le polyélectrolyte est un alginate.

Les alginates sont avantageusement produits à partir d'algues brunes appelées « laminaires ». De tels alginates présentent avantageusement une teneur en masse en α-L-guluronate supérieure à environ 50%, de préférence supérieure à 55%, voire supérieure à 60%.

De préférence, le polyélectrolyte à l'état gélifié est l'alginate de calcium.

Les chaînes individuelles de polyélectrolyte dans l'état liquide présentent avantageusement une masse molaire supérieure à 65 000 g/mol.

Dans l'état gélifié, les chaînes individuelles de polyélectrolyte forment, avec les cations divalents, un réseau tridimensionnel cohérent qui retient le cœur 3 et empêche son écoulement éventuel. Les chaînes individuelles sont retenues les unes par rapport aux autres et ne peuvent pas s'écouler librement les unes par rapport aux autres. Dans cet état, la viscosité du gel formé est infinie.

Le gel tridimensionnel de polyélectrolyte contenu dans les phases externes 5A, 5B emprisonne de l'eau (et au moins un agent tensioactif lorsqu'il est présent). La teneur massique de polyélectrolyte dans les phases externes 5A, 5B est par exemple comprise entre 0,5% et 5% par rapport à la masse totale de l'enveloppe gélifiée 5.

L'agent tensioactif éventuellement contenu dans l'enveloppe gélifiée 5 est avantageusement un tensioactif anionique, un tensioactif non ionique, un tensioactif cationique ou un mélange de ceux-ci. La masse moléculaire de l'agent tensioactif est par exemple comprise entre 150 g/mol et 10 000 g/mol, avantageusement entre 250 g/mol et 1 500 g/mol.

Dans le cas où le tensioactif est un tensioactif anionique, il est par exemple choisi parmi les alkylsulfates, les alkylsulfonates, les alkylarylsulfonates, les alkylphosphates alcalins, les dialkylsulfosuccinates, les sels d'alcalino-terreux d'acides gras saturés ou non. Ces tensioactifs présentent avantageusement au moins une chaîne hydrocarbonée hydrophobe présentant un nombre de carbones supérieur à 5, voire 10 et au moins un groupement anionique hydrophile, tel qu'un sulfate, un sulfonate ou un carboxylate lié à une extrémité de la chaîne hydrophobe.

Dans le cas où le tensioactif est un tensioactif cationique, il est par exemple choisi parmi les sels d'halogénures d'alkylpyridium ou d'alkylammonium comme le chlorure ou le bromure de n-éthyldodecylammonium, le chlorure ou le bromure de cétylammonium (CTAB). Ces tensioactifs présentent avantageusement au moins une chaîne hydrocarbonée hydrophobe présentant un nombre d'atomes de carbone supérieur à 5, voire 10 et au moins un groupement cationique hydrophile, tel qu'un cation d'ammonium quaternaire.

Dans le cas où le tensioactif est un tensioactif non ionique, il est par exemple choisi parmi des dérivés polyoxyéthylénés et/ou polyoxypropylénés des alcools gras, des acides gras, ou des alkylphénols, des arylphénols, ou parmi des alkylglucosides, des polysorbates et des cocamides.

Selon un mode de réalisation de l'invention, l'agent tensioactif est le laurylsulfate de sodium (SLS ou SDS).

La teneur massique en agent tensioactif dans l'enveloppe gélifiée 5 est supérieure à 0,001 % et est avantageusement inférieure à 0,1 %.

### Deuxième mode de réalisation des capsules

En référence à la figure 3, on décrit une série de capsules selon un deuxième mode de réalisation de l'invention. La série comporte des capsules analogues entre elles, aussi seule une capsule 10 de la série sera décrite.

La capsule 10 est analogue à la capsule 1A représentée sur les figures 1 et 2. La capsule 10 est une capsule « simple » ou « complexe » au sens défini ci-dessus, c'est-à-dire qu'elle comporte un cœur (non visible) à une seule phase, ou à plusieurs phases comme cela a été décrit pour la capsule 1A.

Les éléments similaires portent les mêmes références numériques et ne seront pas décrits à nouveau. Seules les différences seront décrites en détail ci-après.

Les portions 7A, 7B ne présentent pas des aires sensiblement égales. Par exemple, l'aire de la portion 7A est inférieure à 1/3, de préférence inférieur à 1/4, de préférence inférieur à 1/6^{e}, de préférence inférieur à 1/8^{e}, de préférence inférieur à 1/10^{e} de préférence inférieur à 1/12^{e} de préférence inférieur à 1/14^{e} de préférence inférieur à 1/16^{e} de préférence inférieur à 1/18^{e} de préférence inférieur à 1/20^{e}, et mieux inférieur à 1/22^{e}, de l'aire totale de la capsule 10.

La portion 7A forme un secteur étroit s'étendant par exemple entre deux points, ou pôles, N, S diamétralement opposés de la surface externe 7.

Avantageusement, l'aire de la portion 7A est inférieure à 1/10^{e}, de préférence inférieur à 1/12^{e} de l'aire totale de la capsule 10.

### Troisième mode de réalisation des capsules

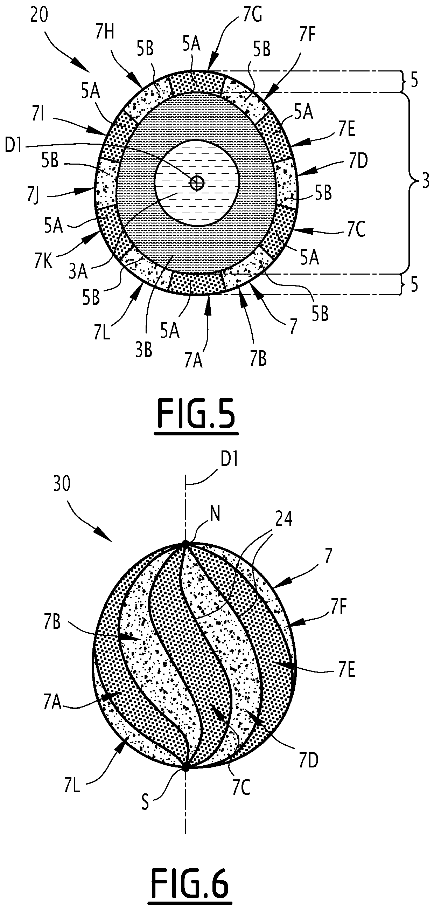

En référence aux figures 4 et 5, on décrit une série de capsules selon un troisième mode de réalisation de l'invention. La série comporte des capsules analogues entre elles, aussi seule une capsule 20 de la série sera décrite.

La capsule 20 est analogue à la capsule 1A représentée sur les figures 1 et 2. Les éléments similaires portent les mêmes références numériques et ne seront pas décrits à nouveau. Seules les différences seront décrites en détail ci-après.

Les deux phases externes 5A, 5B ne sont plus continues sur la surface externe 7 de la capsule 20, et forment une pluralité de portions 7A, 7B, ... 7L distinctes en forme de secteurs (« tranches de melon ») sur cette surface externe.

Dans cet exemple, les secteurs sont au nombre de douze. Les secteurs formés par la phase externe 5A alternent avec les secteurs formés par la phase externe 5B autour d'une direction polaire D1 de la capsule 20.

Les secteurs sont avantageusement sensiblement délimités par des méridiens de la capsule 20, c'est-à-dire par des lignes sensiblement circulaires joignant deux pôles opposés N, S de la capsule 20.

Avantageusement, les secteurs présentent des aires sensiblement égales les unes aux autres (i. e. qui ne diffèrent par exemple pas plus de 5%, voire pas plus de 2,5%, l'une de l'autre).

Selon d'autres variantes non représentées, le nombre de secteurs est différent de douze. Il est par exemple de quatre, six, huit, dix, ou de plus de douze.

Selon une variante, les secteurs présentent des aires différentes les unes des autres.

Selon d'autres variantes encore (non représentée), les secteurs sont formés par plus de deux phases externes, par exemple trois ou quatre, voire plus. L'alternance des phases externes autour de la direction polaire D1 est simple (par exemple 1, 2, 3, 1, 2, 3..., chaque chiffre symbolisant une phase externe distincte), ou en variante suit d'autres motifs plus complexes (par exemple 1, 2, 3, 2, 1, 2, 3, 2, 1...).

Suivant une variante non représentée, le nombre de secteurs est par exemple impair, et peut être avantageusement de trois, cinq, sept, neuf, onze, ou plus.

Selon une autre variante encore (non représentée), les phases externes alternent sans former un motif régulier qui se répète angulairement, c'est-à-dire qu'en fait la période de l'alternance présente un nombre de secteurs égal au nombre total de secteurs (par exemple une capsule à cinq secteurs 1, 2, 3, 1, 2, 1, 2, 3, 1, 2...).

### Variante du troisième mode de réalisation des capsules

En référence à la figure 6, on décrit une série de capsules selon une variante du troisième mode de réalisation de l'invention. La série comporte des capsules analogues entre elles, aussi seule une capsule 30 de la série sera décrite.

La capsule 30 est analogue à la capsule 20 représentée sur les figures 4 et 5. Les éléments similaires portent les mêmes références numériques et ne seront pas décrits à nouveau. Seules les différences seront décrites en détail ci-après.

Les secteurs formés par les portions 7A, 7B, ... 7L ne sont plus délimités par des méridiens de la capsule 30, mais par des lignes courbes 24 (i. e. présentant une torsion) sur la surface externe 7.

Les lignes courbes 24 s'étendent par exemple sensiblement entre le pôle N et le pôle S de la capsule 30.

Chacune des lignes courbes 24 peut être vue comme un méridien déformé d'un côté et/ou de l'autre sur la surface externe 7.

Selon une variante (non représentée), les secteurs formés par les sections 7A, 7B ne sont plus délimités par des méridiens de la capsule 1A, 10, mais par des lignes courbes (i. e. présentant une torsion) sur la surface externe 7, à l'image des courbes 24 susmentionnées.

### Dispositif

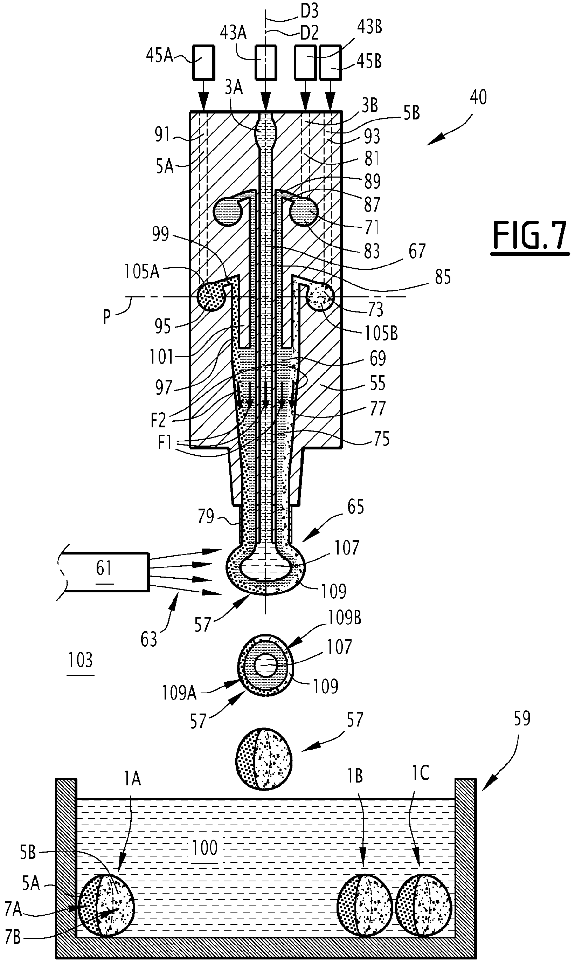

En référence à la figure 7, on décrit un dispositif 40 adapté pour mettre en œuvre un procédé selon l'invention pour produire la capsule 1A et une série de capsules analogues à cette capsule.

Le dispositif 40 comprend des sources 43A, 43B, 45A, 45B fournissant respectivement les phases internes 3A, 3B et les phases externes 5A, 5B à l'état non gélifié, une buse 55 connectée en amont aux sources pour former une série de corps liquides 57 par co-extrusion, et un bain de gélification 59.

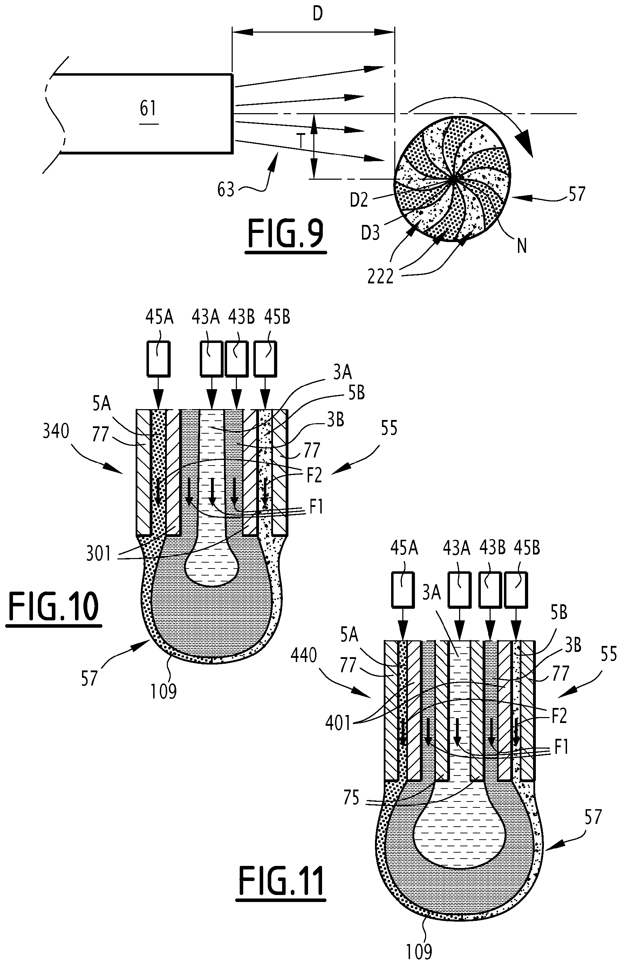

Le dispositif comprend optionnellement un système 61 (figures 7 et 9) pour appliquer un éventuel flux gazeux 63 sur les corps liquides 57 en formation. Il est à noter que le système 61 n'est pas utilisé pour la fabrication des capsules 1A et 10 décrites plus haut.

Le dispositif 40 comprend avantageusement aussi un bain (non représenté) de rinçage et de stockage des capsules produites.

La buse 55 définit un axe D2 de co-extrusion des corps liquide 57, par exemple sensiblement vertical. La buse 55 comporte en aval un embout 65 à l'extrémité duquel se forment les corps liquides 57.

Les termes « amont » et « aval » s'entendent ici par rapport au sens de circulation des phases internes 3A, 3B et des phases externes 5A, 5B dans la buse 55.

La buse 55 délimite un conduit interne 67 s'étendant par exemple selon l'axe D2 pour véhiculer la phase interne 3A, et un conduit externe 69 coaxial avec le conduit interne pour véhiculer la phase interne 3B et les phases externes 5A, 5B. La buse 55 définit aussi un système 71 d'amenée de la phase interne 3B jusqu'au conduit externe 69, et un système d'amenée 73 des phases externes 5A, 5B jusqu'au conduit externe.

Le conduit interne 67 est séparé du conduit externe 69 par une première enveloppe 75, par exemple de forme tubulaire. Le conduit interne 67 est connecté en amont à la source 43A et débouche en aval à l'extrémité de l'embout 65.

Le conduit externe 69 est délimité radialement intérieurement par la première enveloppe 75, et radialement extérieurement par une enveloppe externe 77, de préférence se rétrécissant vers l'aval, et se prolongeant avantageusement en un tube 79, par exemple métallique.

Le conduit externe 69 est connecté en amont aux systèmes 71, 73 et débouche en aval à l'extrémité de l'embout 65.

Le système 71 comprend un canal d'alimentation 81 relié à la source 43B, un collecteur 83, un conduit annulaire 85 coaxial avec le conduit interne 67 et débouchant dans le conduit externe 69, et un conduit de liaison 87 reliant le collecteur 83 au conduit annulaire 85 et créant avantageusement une perte de charge.

Par « collecteur », on entend ici un réservoir présentant un volume suffisant pour que l'écoulement de la phase concernée dans le réservoir soit lent et permette une homogénéisation de la pression de la phase dans le réservoir. Ainsi, l'écoulement à partir de ce réservoir est conditionné par la structure de la buse 55 en aval du réservoir et non par la manière dont le réservoir est alimenté.

Ainsi, grâce à la perte de charge créée par le conduit de liaison 87, il est possible de réguler l'écoulement du flux en aval de la perte de charge et d'homogénéiser le flux injecté dans le conduit annulaire 85 et donc dans le conduit externe 69.

Le conduit annulaire 85 est avantageusement délimité intérieurement par la première enveloppe 75.

Le collecteur 83 est avantageusement de révolution autour de l'axe D2, et par exemple torique.

Le conduit de liaison 87 est par exemple de révolution autour de l'axe D2. Le conduit de liaison 87 s'étend à partir du haut du collecteur 83 vers un coude 89 situé plus haut que le collecteur 83, afin de permettre une évacuation d'éventuelles bulles présentes dans l'écoulement.

Le système 73 est avantageusement analogue au système 71, si ce n'est que, dans cet exemple, il comprend deux canaux d'alimentation 91, 93 connectés respectivement aux sources 45A, 45B. Le système 73 comprend aussi un collecteur 95, un conduit annulaire 97, et un conduit de liaison 99.

Le conduit annulaire 97 débouche dans le conduit externe 69. Le conduit annulaire 97 est radialement plus éloigné de l'axe D2 que le conduit annulaire 85, et est séparé de ce dernier par une enveloppe intermédiaire 101 qui ne se prolonge pas jusqu'à l'embout 65.

Les canaux d'alimentation 91, 93 débouchent par exemple dans des zones diamétralement opposées du collecteur 95.

Le bain de gélification 59 est par exemple situé à l'aplomb de la buse 55.

Le bain de gélification 59 comprend une solution gélifiante 100 comportant au moins un réactif, par exemple des cations Ca²⁺, propre à réagir avec le(s) polyélectrolyte(s) de chacune des phases externes 5A, 5B des corps liquide 57 pour faire passer le(s)dit(s) polyélectrolyte d'un état liquide à un état gélifié.

### Procédé

Un procédé selon l'invention, mis en œuvre dans le dispositif 40, va maintenant être décrit en référence à la figure 7. Le procédé permet de fabriquer la capsule 1A et une série de capsules analogues.

Le procédé comprend une étape de convoyage d'un premier flux F1 des phases internes 3A, 3B, et d'un deuxième flux F2 des deux phases externes 5A, 5B à l'état non gélifié, et une étape de formation successive, à partir du premier flux et du deuxième flux, des corps liquides 57.

Le procédé comprend avantageusement une étape de chute des corps liquide 57 à travers un volume d'air 103, et une étape d'immersion de chaque corps liquide dans le bain de gélification 59 pour obtenir les capsules.

Le procédé comprend une étape optionnelle de rinçage/stockage des capsules.

La source 43A alimente le conduit interne 67 en un flux de phase interne 3A.

La source 43B alimente le collecteur 83 en phase interne 3B via le canal d'alimentation 81. La phase interne 3B se déverse ensuite dans le conduit annulaire 85.

Le flux de phase interne 3A dans le conduit interne 67, et le flux de phase interne 3B dans le conduit annulaire 85 puis dans le conduit externe 69 forment ensemble le premier flux F1. Dans cet exemple, le premier flux F1 comporte deux flux physiquement séparés par la première enveloppe 75.

Le collecteur 95 entoure le premier flux F1 autour d'une direction locale de circulation D3 du premier flux.

La direction locale de circulation D3 est, dans cet exemple, confondue avec l'axe D2 de la buse 55.

Les sources 45A et 45B alimentent le collecteur 95 en phases externe 5A, 5B à l'état non gélifié via les canaux d'alimentation 91, 93. Ceci crée deux zones d'alimentation 105A, 105B distinctes dans le collecteur 95, qui alternent angulairement autour de la direction locale de circulation D3.

Puis, les phases externes 5A, 5B se déversent à partir des zones d'alimentation 105A, 105B de manière homogène dans le conduit annulaire 97, dans lequel elles forment le deuxième flux F2.

Le deuxième flux F2 entoure le premier flux F1. Le deuxième flux F2 est annulaire dans le conduit annulaire 97, puis converge toujours annulairement dans le conduit externe 69, et demeure annulaire au niveau de l'embout 65.

Dans le conduit externe 69, le deuxième flux F2 n'est pas physiquement séparé du premier flux F1, mais ne se mélange pas appréciablement avec le premier flux compte-tenu des vitesses de déplacement des flux F1 et F2 qui sont supérieures à la vitesse de mélange éventuel desdits flux F1 et F2. De même, le deuxième flux F2 comporte un flux de chaque phase externe 5A, 5B qui ne se mélangent pas appréciablement entre eux compte-tenu des vitesses de déplacement de chaque phase externe 5A, 5B qui sont supérieures à la vitesse de mélange éventuel desdites flux phase externe 5A, 5B.

En sortie de la buse 55, la phase interne 3A rentre en contact avec la phase interne 3B et les deux phases externes 5A, 5B rentrent en contact avec le premier flux F1 formé des phases internes 3A, 3B.

Dans l'embout 65, chacune des deux phases externes 5A, 5B est en contact avec le tube 79.

Les corps liquides 57 sont sensiblement sphériques. Chaque corps liquide 57 comprend une goutte 107 formée par les phases internes 3A et 3B, et une pellicule périphérique 109 revêtant la goutte et comportant les deux phases externes 5A, 5B bien séparées.

Pour la fabrication de la capsule 1A, le système 61 optionnel n'est pas utilisé.

La pellicule 109 comporte sur sa surface externe deux portions distinctes 109A, 109B formées respectivement par les deux phases externes 5A, 5B à l'état non gélifié.

Les corps liquides 57 se détachent de la buse 55 avantageusement par gravité, et tombent à travers le volume d'air 103. Le procédé est donc effectué goutte à goutte (i.e. par *dripping* en anglais).

Toutefois, le procédé selon l'invention peut également fonctionner de manière continue par séparation d'un jet (en anglais *jetting*). Selon ce mode de réalisation, le procédé comprend alors :
- une étape de formation en sortie de buse 55 d'un jet formé du flux F2 entourant de manière concentrique le flux F1, puis
- une étape de formation d'une pluralité de corps liquides 57 par fragmentation du jet dans le volume d'air 103 sous l'effet de la gravité.

Lorsque les corps liquides 57 arrivent dans la solution 100, les phases externes 5A, 5B passent à l'état gélifié et les corps liquide 57 deviennent les capsules telles que la capsules 1A, 1B, 1C.

Les débits et viscosités des différentes phases requises pour la fabrication de capsules selon l'invention relèvent des compétences générales de l'homme du métier.

Les débits d'injection en phases externes 5A et 5B dans la buse 55 peuvent être identiques ou différents. Avec des débits identiques, on obtient des capsules dans lesquelles la portion 7A présente une aire sensiblement égale à celle de la portion 7B, comme dans la capsule 1A.

Avec des débits différents, on obtient des aires différentes.

Si le ratio du débit en phase externe 5A divisé par le débit total du deuxième flux F2 est inférieur par exemple à 1/3, à 1/4, à 1/6^{e}, à 1/8^{e}, à 1/10^{e}, à 1/12^{e}, à 1/14^{e}, à 1/16^{e}, à 1/18^{e}, à 1/20^{e}, et de préférence inférieur à 1/22^{e}, on obtient une capsule telle que la capsule 10 représentée sur la figure 3.

Selon un mode de réalisation particulier, les solutions liquides comprenant chacune au moins un polyélectrolyte liquide propre à gélifier peuvent également différer les unes des autres de manière à assurer la formation d'une enveloppe gélifiée 5 qui peut présenter une hétérogénéité mécanique. Cela peut notamment contribuer à créer des points de rupture de ladite enveloppe gélifiée. Cette hétérogénéité mécanique peut notamment résulter :
- du choix des polyélectrolytes, notamment du rapport G (guluronate) / M (mannuronate) lorsque le polyélectrolyte est de l'alginate, et/ou
- des concentrations respectives en polyélectrolytes dans les phases externes 5A, 5B à l'état non gélifié.

### Variante du dispositif

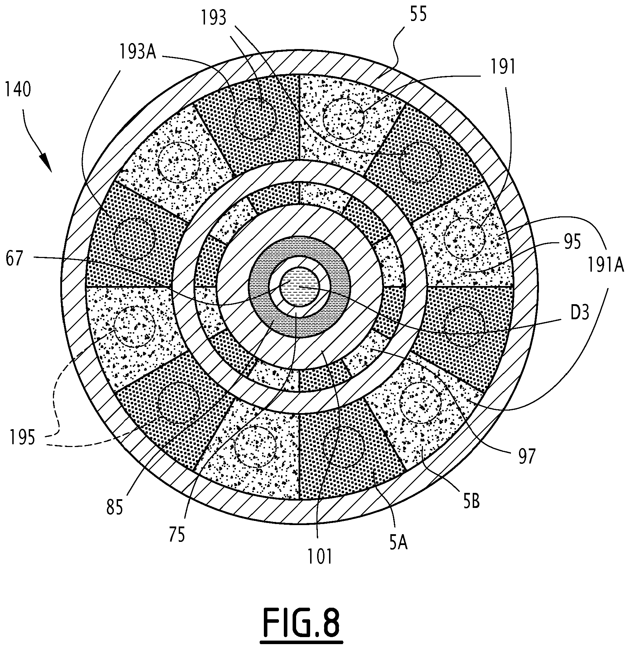

En référence à la figure 8, on décrit un dispositif 140 constituant une variante du dispositif 40. Le dispositif 140 est analogue au dispositif 40 représenté sur la figure 7. Les éléments similaires portent les mêmes références numériques et ne seront pas décrits à nouveau. Seules les différences seront décrites en détail ci-après.

Le dispositif 140 diffère en ce qu'il comporte une pluralité de canaux d'alimentation 191 (dans l'exemple six) reliés à la source 45A, et une pluralité de canaux d'alimentation 193 (dans l'exemple six) reliés à la source 45B. Les canaux peuvent être distincts ou bien résulter de la ramification d'un tronc commun.

Les canaux d'alimentation 191, 193 forment des orifices d'entrée 195 dans le collecteur 95 qui sont angulairement successifs autour de la direction locale de circulation D3.

Ceci crée une pluralité de zones d'alimentation 191A, 193A distinctes dans le collecteur 95, qui alternent angulairement autour de la direction locale de circulation D3.

Il est ainsi possible de fabriquer des capsules telles que la capsule 20 représentée sur les figures 4 et 5.

### Variante du procédé

En référence à la figure 9, selon une variante, le procédé comprend une étape optionnelle d'application d'un flux gazeux 63, en particulier un flux d'air, vers chacun des corps liquides 57 en formation.

Le flux gazeux 63 a une composante tangentielle T par rapport à l'axe D2, ce qui met en rotation les corps liquides 57 en formation et permet par exemple d'obtenir des corps liquides comportant des secteurs 222 préfigurant les secteurs 7A, 7B, ... 7L de la capsule 30 représentée sur la figure 6.

En fonction de l'effet esthétique souhaité, il est possible de jouer sur la pression et/ou le débit d'alimentation en flux gazeux et/ou le diamètre d'éjection du système 61, afin de modifier la vitesse de rotation des corps liquide 57 de façon à obtenir un effet visuel de spirale plus ou moins prononcé.

On peut également :
- adapter l'angle moyen du flux d'air 63 fait avec l'axe D2 de la buse 55, de préférence entre 45° et 135°, et/ou
- adapter la distance D entre la sortie du système 61 et le corps liquide 57 en formation, de préférence entre 2 mm et 15 mm.

La zone de contact entre le flux gazeux 63 et le corps liquide 57 en formation peut se situer au niveau :
(i) d'une zone supérieure,
(ii) d'une zone centrale, et/ou
(iii) d'une zone inférieure,
du corps liquide 57 en formation par rapport à l'axe D2.

La zone de contact se situe de préférence au niveau de la zone centrale.

Le flux gazeux 63 peut comprendre au moins un agent colorant, en particulier choisi dans le groupe comprenant des pigments, des nacres (paillettes), des matériaux à effet optique, en particulier des cristaux liquides, et leurs mélanges, de préférence les pigments et /ou les nacres.

En variante (non représentée), plusieurs flux d'air sont appliqués sur les corps liquides 57, en particulier avec :
- des orientations différentes, le cas échéant positionnés à des hauteurs différentes selon l'axe D2 pour réaliser des spires qui changent de trajectoire dans la direction polaire D1 des capsules, et/ou
- au moins un flux d'air, voire chaque flux d'air, comprend en outre au moins un agent colorant, les colorants étant différents ou au moins dans des teneurs différentes,

En outre, il est possible de modifier la saturation en humidité du flux gazeux 63 pour éviter un dessèchement de la pellicule 109, notamment sur le bout de la buse 55 (i. e. au niveau de l'extrémité de l'embout 65).

La pression d'alimentation en air en sortie du système 61 est avantageusement ajustée pour obtenir une vitesse du flux d'air 63 au niveau du corps liquide 57 en formation suffisante pour obtenir l'effet de rotation escompté, et inférieure à une limite supérieure à partir de laquelle un arrachage non souhaité du corps liquide 57 en formation peut se produire. En particulier, cette pression relative est inférieure à 100kPa (1bar), de préférence inférieure à 50 kPa (0,5 bar).

En particulier, cette pression relative est comprise entre 5000 Pa (50 millibars) et 90 kPa (900 millibars), de préférence entre 10 kPa et 70 kPa (100 millibars et 700 millibars), et mieux entre 20 kPa et 40 kPa (200 millibars et 400 millibars).

Avantageusement, lorsque le procédé selon l'invention comprend l'application du flux gazeux 63, les débits des différentes phases sont réduits dans la buse 55. Ainsi, le corps liquide 57 en formation dispose d'un temps plus important pour tourner, ce qui a un impact sur l'intensité du motif spiralé formé. De préférence, les débits des différentes phases sont réduits d'environ 50 % par rapport aux mêmes délits dans un procédé sans application d'un flux gazeux 63.

### Autres variantes du dispositif

En référence à la figure 10, on décrit un dispositif 340 constituant une variante du dispositif 40. Le dispositif 340 est analogue au dispositif 40 représenté sur la figure 7. Les éléments similaires portent les mêmes références numériques et ne seront pas décrits à nouveau. Seules les différences seront décrites en détail ci-après.

La buse 55 du dispositif 340 ne comporte pas de première enveloppe 75 qui séparerait physiquement les phases internes 3A et 3B jusqu'à la sortie de la buse. En revanche, la buse 55 comporte une enveloppe intermédiaire 301 séparant physiquement le deuxième flux F2 du premier flux F1 jusqu'à la sortie.

En référence à la figure 11, on décrit un dispositif 440 constituant une autre variante du dispositif 40. Le dispositif 440 est analogue au dispositif 40. Les éléments similaires portent les mêmes références numériques et ne seront pas décrits à nouveau. Seules les différences seront décrites en détail ci-après.

La buse 55 du dispositif 440 comporte une enveloppe intermédiaire 401 séparant physiquement le deuxième flux F2 du premier flux F1 jusqu'à la sortie. Ainsi, la buse 55 comporte trois enveloppes séparant la phase interne 3A, la phase interne 3B, et le deuxième flux F2.

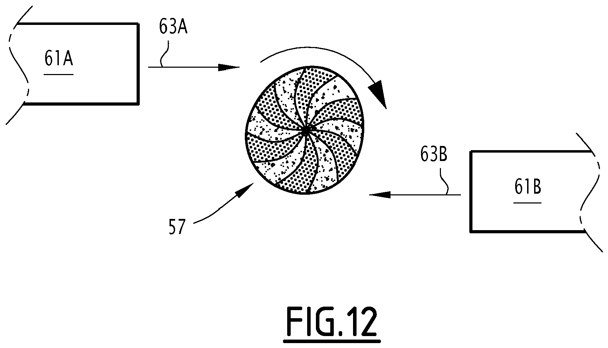

Selon une variante représentée sur la figure 12, le système 61 comprend deux sous-systèmes 61A, 61B pour appliquer respectivement deux flux gazeux 63A, 63B sur les corps liquides 57 en formation.

Chacun des sous-systèmes 61A, 61B comprend par exemple une buse, les buses étant agencées pour que les flux gazeux 63A, 63B soient opposés l'un à l'autre et sensiblement tangents à la surface des corps liquides 57 en formation.

Les points d'impact des flux gazeux 63A, 63B sur les corps liquides 57 sont par exemple diamétralement opposés l'un à l'autre.

Ceci permet d'améliorer le décrochage des corps liquides 57 de la buse 55 et permet d'obtenir un effet de spirale plus important sur une capsule telle que la capsule 30 représentée sur la figure 6.

### Composition cosmétique et procédé de traitement cosmétique

Selon un mode de réalisation, la composition de l'invention est une composition cosmétique en association avec milieu physiologiquement acceptable ou un milieu de réception cosmétiquement acceptable. Par "milieu physiologiquement acceptable" pou « un milieu de réception cosmétiquement acceptable », on entend désigner un milieu convenant particulièrement à l'application d'une composition de l'invention sur les matières kératiniques, notamment la peau, les lèvres, les ongles, les cils ou les sourcils, et de préférence la peau.

Le milieu physiologiquement acceptable est généralement adapté à la nature du support sur lequel doit être appliquée la composition, ainsi qu'à l'aspect sous lequel la composition doit être conditionnée. En particulier, ledit milieu présente une viscosité suffisante pour assurer une suspension des capsules et ainsi éviter tout phénomène de sédimentation ou de crémage desdites capsules dans ledit milieu. En effet, pour des raisons évidentes, le caractère suspensif du milieu peut participer à renforcer le visuel attractif et inédit des capsules selon l'invention.

La présente invention a également pour objet un procédé de traitement cosmétique non thérapeutique de la peau comprenant une étape d'application sur la peau d'au moins une couche de la composition selon l'invention.

Ce procédé est de préférence mis en œuvre avec une composition comprenant des capsules dont le ou les agents actifs sont des actifs cosmétiques non thérapeutiques.

Il peut notamment s'agir d'une composition cosmétique.

L'invention a également pour objet l'utilisation cosmétique de la composition cosmétique décrite précédemment.

Le milieu de réception des capsules est par exemple une composition aqueuse.

La composition aqueuse peut être liquide ou gélifiée.

Lorsqu'elle est gélifiée, la composition aqueuse est sous forme de « gel aqueux », c'est-à-dire qu'il s'agit d'une solution comprenant de l'eau et un gélifiant.

Dans le cadre de la présente description, on entend par « gélifiant » un composé propre à donner à une composition la consistance d'un gel.

Le gélifiant est de préférence choisi dans le groupe constitué des polyosides, des galactomannanes, des polysaccharides, des glycosaminoglycanes, des polyols et leurs mélanges.

Avantageusement, il est choisi dans le groupe constitué de la gomme xanthane, du carraghénane, de la caroube, de la gomme guar, de la gellane, de l'acide hyaluronique, du glycérol, du propanediol, de la cellulose ou de ses dérivés, et de leurs mélanges.

De préférence, la composition aqueuse possède une viscosité inférieure à 50 Pa.s telle que mesurée à 25°C, de préférence inférieure à 20 Pa.s. Avantageusement, la composition aqueuse (A) possède une viscosité comprise de 2 Pa.s à 15 Pa.s telle que mesuré à 25°C.

Une telle viscosité du gel aqueux permet une bonne suspension des capsules, notamment sur une durée d'au moins un mois, à une température de 40°C.

Selon une variante, la composition ne suspend pas les capsules, c'est-à-dire que celles-ci sédimentent dans la composition (A). Pour cela, on peut notamment utiliser une composition (A) de viscosité inférieure à 2 Pa.s.

Avantageusement, le gel aqueux est transparent afin que le consommateur puisse visualiser la capsule. Sa texture est choisie en fonction de la texture que l'on désire obtenir pour la composition de l'invention.

La viscosité est mesurée par la méthode suivante, notamment décrite dans la demande internationale WO 2013/132082 au nom de la Demanderesse.

De préférence, le pourcentage massique d'eau du gel aqueux est d'au moins 70%, notamment compris de 70% à 85%, préférentiellement compris de 70% à 80%, par rapport à la masse totale de la composition.

La composition aqueuse peut également comprendre un agent cosmétique, dermo-pharmaceutique, pharmaceutique, parfumant ou alimentaire tel que défini ci-dessus.

La composition aqueuse peut également comprendre un agent conservateur, un agent colorant, en particulier des pigments, et/ou des nacres.

Typiquement, le rapport massique entre la composition aqueuse et les capsules est compris de 30/70 à 70/30, de préférence de 40/60 à 60/40.

### Avantages

Grâce aux caractéristiques décrites ci-dessus, l'invention fournit des compositions présentant un aspect visuel inédit, dérivant de la mise en œuvre de capsules dotées d'une structure particulière, de préférence monodisperses, peu coûteuse à fabriquer et adaptée pour préserver l'intégrité des actifs encapsulés (i.e. réduire, voire prévenir, leur vieillissement consécutif, notamment, d'une oxydation, d'une dégradation enzymatique et/ou ionique, etc.) tout en étant adaptée à de multiples usages, notamment dans le domaine cosmétique, voire pharmaceutique ou agroalimentaire.

L'enveloppe gélifiée 5 ayant en surface 7 au moins deux portions 7A, 7B distinctes formées respectivement par les deux phases externes 5A, 5B dont au moins l'une comporte au moins un agent colorant, l'invention a pour effet technique que les capsules possèdent au moins deux zones qui sont perçues comme distinctes par un utilisateur, et ce plus directement qu'avec les capsules de l'art antérieur.

Dans certains modes de réalisation, l'existence de ces zones confère aux capsules de nouvelles propriétés chimiques, grâce à l'incorporation de produits spécifiques dans une ou plusieurs des phases distinctes de l'enveloppe gélifiée 5.

L'invention fournit donc un système d'encapsulation d'actifs doté d'un aspect visuel inédit et assurant une libération/diffusion contrôlée des actifs encapsulés.

De plus, grâce à l'invention, il est possible de créer au moins une zone de faiblesse des capsules. Par exemple, une des phases de l'enveloppe gélifiée 5 peut avantageusement comporter un taux en polyélectrolyte gélifié, en particulier d'alginate, inférieur à celui des autres phases de l'enveloppe, ce qui la rend localement moins résistante mécaniquement. Cette phase peut avantageusement former une strie à la surface de l'enveloppe gélifiée 5 et constituer ainsi une ligne de faiblesse de la capsule.

L'invention fournit aussi un procédé compétitif pour arriver à ces résultats.

Dans toute la présente description, y compris les revendications, l'expression « comprenant un » doit être comprise comme étant synonyme de « comprenant au moins un », sauf si le contraire est spécifié.

Les expressions « compris entre ... et ... », « compris de ... à ... » et « allant de ... à ... » doivent se comprendre bornes incluses, sauf si le contraire est spécifié.

Les quantités des ingrédients figurant dans les exemples sont exprimées en pourcentage en poids par rapport au poids total de la composition, sauf indication contraire.

Les exemples qui suivent illustrent la présente invention sans en limiter la portée.

### Exemples

### Dispositif expérimental

Dans les deux exemples ci-après, le procédé de préparation de capsules, telles que la capsule 1A, est basé sur la co-extrusion concentrique de compositions (ou phases) via un dispositif millifluidique à triple enveloppe, tel que le dispositif 440 représenté sur la figure 11, pour former des gouttes multi-composante telles que les corps liquides 57.

Les compositions des phases internes 3A et 3B, et des phases externes 5A et 5B pour la fabrication de capsules sont décrites dans le tableau ci-dessous en % massique.

| **Phase** | **Dénomination commerciale** | **Fournisseur** | **INCI** | **%** | **% / capsu**le |
|---|---|---|---|---|---|
| Phase interne 3A | Huile végétale raffinée de novau d'abricot | Ardex | PRUNUS ARMENIACA KERNEL OIL | 100,00 | 16,67 |
| | **Total** | | | **100,00** | **16,67** |
| Phase interne 3B | Eau osmosée | - | AQUA | qsp | qsp |
| | Microcare PE | Thor | PHENOXYETHANOL, AQUA | 0,80 | 0,53 |
| | Microcare emollient PTG | Thor | PENTYLENE GLYCOL, AQUA | 2,00 | 1,33 |
| | Protanal LF 200 FTS | FMC Bio polymère | ALGIN | 0,03 | 0,02 |
| | Phylcare sodium hyaluronate CPS | Biophil | SODIUM HYALURONATE | 0,95 | 0,63 |
| | **Total** | | | **100,00** | **66,67** |
| Phase externe 5A | Eau osmosée | - | AQUA | 92,06 | 14,11 |
| | Microcare PE | Thor | PHENOXYETHANOL, AQUA | 0,80 | 0,12 |
| | Microcare emollient PTG | Thor | PENTYLENE GLYCOL, AQUA | 2,00 | 0,31 |
| | Protanal LF 200 FTS | FMC Bio polymère | ALGIN | 2,00 | 0,31 |
| | Sodium Dodecyl sulfate PRS Codex | Panréac | SODIUM LAURYL SULFATE | 0,14 | 0,02 |
| | Sunshine Soft **White** | Sunchemical | SYNTHETIC FLUORPHLOGOPITE (AND) TITANIUM DIOXIDE | 3,00 | 0,46 |
| | | | | **100,00** | **15,33** |
| Phase externe 5B | Eau osmosée | - | AQUA | 92,06 | 1,23 |
| | Microcare PE | Thor | PHENOXYETHANOL, AQUA | 0,80 | 0,01 |
| | Microcare emollient PTG | Thor | PENTYLENE GLYCOL, AQUA | 2,00 | 0,03 |
| | Protanal LF 200 FTS | FMC Bio polymère | ALGIN | 2,00 | 0,03 |
| | Sodium Dodecyl sulfate PRS Codex | Panréac | SODIUM LAURYL SULFATE | 0,14 | 0,002 |
| | Colorona mica **black** | Merck | | 3,00 | 0,04 |
| | **Total** | | | **100,00** | **1,33** |

### Formation de capsules gélifiées

A la sortie de la triple enveloppe, se forme alors une goutte multi-composante, le premier flux F1, formé des phases internes 3A, 3B, constituant le cœur et le deuxième flux F2 constituant l'enveloppe externe liquide de la goutte multi-composante. Le cœur est plus particulièrement constitué d'une goutte interne, formée par la phase interne 3A, dans une enveloppe intermédiaire, formée par la phase interne 3B.

La taille de la goutte interne, l'épaisseur de l'enveloppe intermédiaire et de l'enveloppe externe des capsules formées sont contrôlées par l'utilisation de plusieurs pousse-seringues indépendants, réglant les débits d'injection des différentes compositions formant les phases internes 3A, 3B et les phases externes 5A, 5B.

Chaque goutte multi-composante se détache de la triple enveloppe et chute dans un volume d'air, avant d'être immergée dans une solution gélifiante de calcium concentrée à 1M.

Une fois l'enveloppe externe gélifiée, les capsules gélifiées formées sont rincées dans une solution de rinçage à base d'eau.

### Exemple 1 :

Le dispositif millifluidique mis en œuvre est une buse à trois voies coaxiales, avec un collecteur 95 tel que représenté sur la figure 8.

Les débits des différentes phases sont les suivants :
- phase interne 3A : 10 ml/h,
- phase interne 3B : 40 ml/h,
- phase externe 5A : 9,2 ml/h, et
- phase externe 5B : 0,8 ml/h.

Le procédé selon l'invention permet d'obtenir des capsules de taille uniforme présentant un aspect visuel inédit. En effet, chaque capsule comprend douze portions 7A à 7L formant des secteurs, les secteurs de même couleur étant opposés deux à deux et les secteurs blancs étant beaucoup plus larges que les secteurs noirs (sensiblement dans le rapport des débits des phases externes 5A et 5B).

### Exemple 2 : avec application d'un flux d'air

Le dispositif millifluidique mis en œuvre est une buse à trois voies coaxiales dans laquelle le collecteur 95 est alimenté en phases externes 5A, 5B uniquement par deux canaux principaux, comme dans le dispositif 40 représenté sur la figure 7.

Les débits des différentes phases sont les suivants :
- phase interne 3A : 5 ml/h,
- phase interne 3B : 20 ml/h,
- phase externe 5A : 4,6 ml/h, et
- phase externe 5B : 0,4 ml/h.

Le procédé mis en œuvre comprend en outre l'application du flux d'air 63 dirigé vers la zone de contact entre la goutte en formation en sortie de la triple enveloppe, l'application étant réalisée de façon tangente à un côté de la goutte en formation en sortie de dispositif 40.

Le flux d'air 63 présente les caractéristiques suivantes :
- pression en air : 200 mbar
- diamètre interne du tube d'alimentation en air : 0,9 mm,
- angle par rapport à l'axe D2 vertical : 90°,
- distance entre la sortie du tube d'alimentation en air et la pellicule 109 de la goutte en formation : 8 mm.

Le flux d'air 63 entraîne une rotation de la goutte en formation en sortie du dispositif millifluidique par rapport à l'axe vertical D2 dudit dispositif (perpendiculairement au sens d'expansion de la goutte).

Le procédé selon l'invention permet d'obtenir des capsules de taille uniforme et dotées d'un aspect visuel inédit. En effet, chaque capsule comprend deux portions 7A, 7B, la portion 7A blanche étant très large, et la portion 7B noire étant très étroite (comme sur la capsule 10 représenté sur la figure 3) et formant un motif spiralé sur toute l'enveloppe gélifiée 5 des capsules (comme sur la capsule 30 représentée sur la figure 6).

Le dispositif millifluidique mis en œuvre est une buse à trois voies coaxiales, avec un collecteur 95 tel que représenté sur la figure 8.

Les débits des différentes phases sont les suivants :
- phase interne 3A : 10 ml/h,
- phase interne 3B : 40 ml/h,
- phase externe 5A : 9,2 ml/h, et
- phase externe 5B : 0,8 ml/h.

Le procédé selon l'invention permet d'obtenir des capsules de taille uniforme présentant un aspect visuel inédit. En effet, chaque capsule comprend douze portions 7A à 7L formant des secteurs, les secteurs de même couleur étant opposés deux à deux et les secteurs blancs étant beaucoup plus larges que les secteurs noirs (sensiblement dans le rapport des débits des phases externes 5A et 5B).

### Exemple 2 : avec application d'un flux d'air

Le dispositif millifluidique mis en œuvre est une buse à trois voies coaxiales dans laquelle le collecteur 95 est alimenté en phases externes 5A, 5B uniquement par deux canaux principaux, comme dans le dispositif 40 représenté sur la figure 7.

Les débits des différentes phases sont les suivants :
- phase interne 3A : 5 ml/h,
- phase interne 3B : 20 ml/h,
- phase externe 5A : 4,6 ml/h, et
- phase externe 5B : 0,4 ml/h.

Le procédé mis en œuvre comprend en outre l'application du flux d'air 63 dirigé vers la zone de contact entre la goutte en formation en sortie de la triple enveloppe, l'application étant réalisée de façon tangente à un côté de la goutte en formation en sortie de dispositif 40.

Le flux d'air 63 présente les caractéristiques suivantes :
- pression en air : 200 mbar
- diamètre interne du tube d'alimentation en air : 0,9 mm,
- angle par rapport à l'axe D2 vertical : 90°,
- distance entre la sortie du tube d'alimentation en air et la pellicule 109 de la goutte en formation : 8 mm.

Le flux d'air 63 entraîne une rotation de la goutte en formation en sortie du dispositif millifluidique par rapport à l'axe vertical D2 dudit dispositif (perpendiculairement au sens d'expansion de la goutte).

Le procédé selon l'invention permet d'obtenir des capsules de taille uniforme et dotées d'un aspect visuel inédit. En effet, chaque capsule comprend deux portions 7A, 7B, la portion 7A blanche étant très large, et la portion 7B noire étant très étroite (comme sur la capsule 10 représenté sur la figure 3) et formant un motif spiralé sur toute l'enveloppe gélifiée 5 des capsules (comme sur la capsule 30 représentée sur la figure 6).

## Revendications

1. Procédé de fabrication d'une série de capsules (1A ; 10 ; 20 ; 30), chaque capsule (1A ; 10 ; 20 ; 30) comprenant un cœur (3) liquide ou au moins en partie gélifié ou au moins en partie thixotrope, et une enveloppe gélifiée (5) encapsulant totalement le cœur (3) et comportant au moins deux phases externes (5A, 5B), chaque phase externe (5A, 5B) contenant au moins un polyélectrolyte gélifié, l'enveloppe gélifiée (5) ayant une surface externe (7) comportant au moins deux portions (7A, 7B) distinctes formées respectivement par les deux phases externes (5A, 5B), le procédé comprenant les étapes suivantes :
- convoyage d'un premier flux (F1) d'une ou plusieurs phases internes (3A, 3B) destinée(s) à former le cœur (3), et d'un deuxième flux (F2) d'au moins les deux phases externes (5A, 5B) destinées à former l'enveloppe gélifiée (5), chaque phase externe contenant le polyélectrolyte liquide propre à gélifier, au moins l'une des deux phases externes (5A, 5B) comportant au moins un agent colorant, le deuxième flux (F2) entourant le premier flux autour d'un axe (D2) ;
- formation successive, à partir du premier flux (F1) et du deuxième flux (F2), d'une pluralité de corps liquides (57), chaque corps liquide (57) comprenant une goutte (107) de la ou des phases internes (3A, 3B), et une pellicule (109) périphérique revêtant la goutte et comportant les deux phases externes (5A, 5B), la pellicule (109) comportant sur sa surface externe au moins deux portions (109A, 109B) distinctes formées respectivement par les deux phases externes (5A, 5B) ; et
- immersion de chaque corps liquide (57) dans une solution gélifiante (100) contenant un réactif propre à réagir avec le polyélectrolyte de chacune des deux phases externes (5A, 5B) pour former l'une des capsules (1A ; 10 ; 20 ; 30), et récupération de la pluralité de capsules (1A ; 10 ; 20 ; 30).

2. Procédé selon la revendication 1, comportant en outre l'application d'un flux gazeux (63) vers chacun des corps liquides (57) en formation pour déformer lesdites deux portions (109A, 109B) de la surface externe de la pellicule (109) par rotation autour de l'axe (D2), le flux gazeux ayant une composante tangentielle (T) par rapport à l'axe (D2).

3. Procédé selon la revendication 1 ou 2, dans lequel, durant l'étape de formation des corps liquides (57), les deux phases externes (5A, 5B) sont en contact avec le premier flux (F1).

4. Procédé selon l'une quelconque des revendications 1 à 3, dans lequel :
- le convoyage est réalisé dans au moins une buse (55) définissant l'axe (D2), chacune des deux phases externes (5A, 5B) étant en contact avec une enveloppe externe (77) de la buse (55) en sortie de la buse (55), et
- les corps liquides (57) sont formés à une sortie de la buse (55), le procédé comportant une étape de détachement des corps liquides (57) à l'écart de la buse (55).

5. Procédé selon la revendication 4, dans lequel l'étape de convoyage comporte :
- une sortie du deuxième flux (F2) d'au moins un collecteur (95) formé par la buse (55), le collecteur (95) entourant le premier flux (F1) autour d'une direction locale de circulation (D3) du premier flux (F1), et
- une alimentation du collecteur (95) à partir d'au moins deux sources (45A, 45B) fournissant les deux phases externes (5A, 5B) pour créer une pluralité de zones d'alimentation (105A, 105B) distinctes dans le collecteur (95), chacune des zones d'alimentation (105A, 105B) étant remplie par l'une ou l'autre des deux phases externes (5A, 5B), les zones d'alimentation (105A, 105B) remplies par l'une alternant avec celles remplies par l'autre autour de la direction locale de circulation (D3).

6. Procédé selon la revendication 5, dans lequel la buse (55) forme une pluralité de canaux d'alimentation (91, 93) du collecteur (95), chaque canal d'alimentation (91, 93) étant relié à l'une des deux sources (45A, 45B) et formant un orifice d'entrée dans le collecteur (95), les orifices d'entrée étant angulairement successifs autour de la direction locale de circulation (D3).

7. Procédé selon l'une quelconque des revendications 5 ou 6, dans lequel l'une des zones d'alimentation (105A, 105B) est alimentée par un premier débit en l'une ou l'autre des deux phases externes (5A, 5B), et une autre des zones d'alimentation (105A, 105B) est alimentée par un deuxième débit en l'une ou l'autre des deux phases externes (5A, 5B), le premier débit étant inférieur au deuxième débit.

8. Procédé selon la revendication 7, dans lequel le ratio du premier débit divisé par le débit total du deuxième flux (F2) est inférieur à 1/3, de préférence inférieur à 1/4, voire inférieur à 1/6^{e}, en particulier inférieur à 1/8^{e}, et mieux inférieur à 1/10^{e}.

9. Procédé selon l'une quelconque des revendications 4 à 8, dans lequel l'étape de convoyage comprend une séparation physique du premier flux (F1) et du deuxième flux (F2) jusqu'à la sortie de la buse (55) par une enveloppe (301 ; 401) de la buse (55).

10. Série de capsules (1A ; 10 ; 20 ; 30) susceptible d'être fabriquée par un procédé selon l'une quelconque des revendications 1 à 9, dans laquelle chaque capsule (1A ; 10 ; 20 ; 30) comprend :
- un cœur (3) liquide ou au moins en partie gélifié ou au moins en partie thixotrope, le cœur (3) comportant une ou plusieurs phases interne(s) (3A, 3B), et
- une enveloppe gélifiée (5) encapsulant totalement le cœur (3) et comportant au moins deux phases externes (5A, 5B), chaque phase externe contenant un polyélectrolyte gélifié, au moins l'une des deux phases externes (5A, 5B) comportant un agent colorant, l'enveloppe gélifiée (5) ayant une surface externe comportant au moins deux portions (7A, 7B) distinctes formées respectivement par les deux phases externes (5A, 5B).

11. Série de capsules (1A ; 10 ; 20 ; 30) selon la revendication 10, dans laquelle, pour chaque capsule (1A ; 10 ; 20 ; 30), la surface externe (7) de l'enveloppe gélifiée (5) comprend une pluralité de portions (7A, 7B) formées par l'une ou l'autre des deux phases externes (5A, 5B), les portions (7A, 7B) formant des secteurs alternant autour d'une direction polaire (D1) de la capsule (1A ; 10 ; 20 ; 30).

12. Série de capsules (1A ; 10 ; 20 ; 30) selon la revendication 11, dans laquelle les secteurs s'étendent sensiblement entre un pôle (N) de la capsule (20 ; 30) et un pôle opposé (S) selon la direction polaire (D1), les secteurs étant sensiblement délimités :
- soit par des méridiens de la capsule (20 ; 30),
- soit par des lignes courbes (24) s'étendant sensiblement entre le pôle (N) et le pôle (S) opposé.

13. Série de capsules (1A; 10; 20; 30) selon l'une quelconque des revendications 10 à 12, dans laquelle les deux phases externes (5A, 5B) de l'enveloppe gélifiée (5) sont en contact avec le cœur (3).

14. Composition, notamment cosmétique, comprenant une série de capsules (1A ; 10 ; 20 ; 30) selon l'une quelconque des revendications 10 à 13, et un milieu de réception de ces capsules (20 ; 30).

15. Procédé non thérapeutique de traitement cosmétique d'une matière kératinique, en particulier de la peau, comprenant au moins une étape d'application sur ladite matière kératinique d'au moins une composition selon la revendication 14.

## Patentansprüche

1. Verfahren zur Herstellung einer Reihe von Kapseln (1A; 10; 20; 30), wobei jede Kapsel (1A; 10; 20; 30) einen flüssigen oder mindestens teilweise gelierten oder mindestens teilweise thixotropen Kern (3) und eine gelierte Hülle (5) umfasst, die den Kern (3) vollständig einkapselt und mindestens zwei äußere Phasen (5A, 5B) aufweist, wobei jede äußere Phase (5A, 5B) mindestens einen gelierten Polyelektrolyten enthält, wobei die gelierte Hülle (5) eine äußere Fläche (7) hat, die mindestens zwei getrennte Bereiche (7A, 7B) aufweist, die jeweils von den beiden äußeren Phasen (5A, 5B) gebildet werden, wobei das Verfahren die folgenden Schritte umfasst:
- Fördern eines ersten Stroms (F1) einer oder mehrerer innerer Phasen (3A, 3B), die dazu bestimmt sind, den Kern (3) zu bilden, und eines zweiten Stroms (F2) mindestens zweier äußerer Phasen (5A, 5B), die dazu bestimmt sind, die gelierte Hülle (5) zu bilden, wobei jede äußere Phase den zum Gelieren geeigneten flüssigen Polyelektrolyten enthält und mindestens eine der beiden äußeren Phasen (5A, 5B) mindestens einen Farbstoff aufweist, wobei der zweite Strom (F2) den ersten Strom um eine Achse (D2) umgibt;
- aufeinanderfolgendes Bilden einer Vielzahl von Flüssigkeitskörpern (57) aus dem ersten Strom (F1) und dem zweiten Strom (F2), wobei jeder Flüssigkeitskörper (57) einen Tropfen (107) der inneren Phase oder Phasen (3A, 3B) und einen umfänglichen Film (109) umfasst, der den Tropfen umhüllt und die beiden äußeren Phasen (5A, 5B) aufweist, wobei der Film (109) auf seiner äußeren Fläche mindestens zwei getrennte Bereiche (109A, 109B) aufweist, die jeweils von den beiden äußeren Phasen (5A, 5B) gebildet werden; und
- Eintauchen jedes Flüssigkeitskörpers (57) in eine Gelierlösung (100), die ein Reagenz enthält, das geeignet ist, mit dem Polyelektrolyten jeder der beiden äußeren Phasen (5A, 5B) zu reagieren, um eine der Kapseln (1A; 10; 20; 30) zu bilden, und Gewinnen der Vielzahl von Kapseln (1A; 10; 20; 30).

2. Verfahren nach Anspruch 1, außerdem das Zuführen eines Gasstroms (63) zu jedem der in Bildung befindlichen Flüssigkeitskörper (57) aufweisend, um die beiden Bereiche (109A, 109B) der äußeren Fläche des Films (109) durch Drehung um die Achse (D2) zu verformen, wobei der Gasstrom eine tangentiale Komponente (T) in Bezug auf die Achse (D2) aufweist.

3. Verfahren nach Anspruch 1 oder 2, bei dem während des Schritts des Bildens der Flüssigkeitskörper (57) die beiden äußeren Phasen (5A, 5B) in Kontakt mit dem ersten Strom (F1) sind.

4. Verfahren nach einem beliebigen der Ansprüche 1 bis 3, bei dem:
- das Fördern in mindestens einer die Achse (D2) definierenden Düse (55) durchgeführt wird, wobei jede der beiden äußeren Phasen (5A, 5B) am Ausgang der Düse (55) mit einem Außenmantel (77) der Düse (55) in Kontakt ist, und
- die Flüssigkeitskörper (57) an einem Auslass der Düse (55) gebildet werden, wobei das Verfahren einen Schritt des Lösens der Flüssigkeitskörper (57) von der Düse (55) umfasst.

5. Verfahren nach Anspruch 4, wobei der Schritt des Förderns aufweist:
- einen Austritt des zweiten Stroms (F2) aus mindestens einem von der Düse (55) gebildeten Sammler (95), wobei der Sammler (95) den ersten Strom (F1) um eine lokale Strömungsrichtung (D3) des ersten Stroms (F1) herum umgibt, und
- Versorgen des Sammlers (95) aus mindestens zwei Quellen (45A, 45B), die die beiden externen Phasen (5A, 5B) liefern, um eine Mehrzahl von unterschiedlichen Versorgungszonen (105A, 105B) in dem Sammler (95) zu erzeugen, wobei jede der Versorgungszonen (105A, 105B) durch die eine oder die andere der beiden externen Phasen (5A, 5B) gefüllt wird, wobei die Versorgungszonen (105A, 105B), die durch die eine gefüllt werden, sich mit denen abwechseln, die durch die andere um die lokale Strömungsrichtung (D3) gefüllt werden.

6. Verfahren nach Anspruch 5, bei dem die Düse (55) eine Mehrzahl von Kanälen (91, 93) zum Versorgen des Sammlers (95) bildet, wobei jeder Versorgungskanal (91, 93) mit einer der beiden Quellen (45A, 45B) verbunden ist und eine Einlassöffnung in den Sammler (95) bildet, wobei die Einlassöffnungen um die lokale Strömungsrichtung (D3) herum winklig aufeinanderfolgend angeordnet sind.

7. Verfahren nach einem beliebigen der Ansprüche 5 oder 6, bei dem eine der Versorgungszonen (105A, 105B) mit einer ersten Durchflussrate mit einer der beiden externen Phasen (5A, 5B) versorgt wird und eine andere der Versorgungszonen (105A, 105B) mit einer zweiten Durchflussrate mit der einen oder der anderen der beiden externen Phasen (5A, 5B) versorgt wird, wobei die erste Durchflussrate kleiner als die zweite Durchflussrate ist.

8. Verfahren nach Anspruch 7, bei dem das Verhältnis der ersten Durchflussrate dividiert durch die Gesamtdurchflussrate des zweiten Stroms (F2) weniger als 1/3, vorzugsweise weniger als 1/4, oder sogar weniger als 1 /6, insbesondere weniger als 1/8 und besser weniger als 1/10 beträgt.

9. Verfahren nach einem beliebigen der Ansprüche 4 bis 8, bei dem der Förderschritt eine physische Trennung des ersten Stroms (F1) und des zweiten Stroms (F2) bis zum Auslass der Düse (55) durch einen Mantel (301; 401) der Düse (55) umfasst.

10. Reihe von Kapseln (1A; 10; 20; 30), herstellbar durch ein Verfahren nach einem der Ansprüche 1 bis 9, wobei jede Kapsel (1A; 10; 20; 30) umfasst:
- einen flüssigen oder zumindest teilweise gelierten oder zumindest teilweise thixotropen Kern (3), wobei der Kern (3) eine oder mehrere innere Phasen (3A, 3B) umfasst, und
- eine gelierte Hülle (5), die den Kern (3) vollständig einkapselt und mindestens zwei äußere Phasen (5A, 5B) aufweist, wobei jede äußere Phase einen gelierten Polyelektrolyten enthält, wobei mindestens eine der beiden äußeren Phasen (5A, 5B) einen Farbstoff aufweist, wobei die gelierte Hülle (5) eine äußere Fläche hat, die mindestens zwei getrennte Bereiche (7A, 7B) aufweist, die jeweils von den beiden äußeren Phasen (5A, 5B) gebildet werden.

11. Reihe von Kapseln (1A; 10; 20; 30) nach Anspruch 10, bei der für jede Kapsel (1A; 10; 20; 30) die äußere Fläche (7) der gelierten Hülle (5) eine Mehrzahl von Bereichen (7A, 7B) umfasst, die durch die eine oder andere der beiden äußeren Phasen (5A, 5B) gebildet werden, wobei die Bereiche (7A, 7B) Sektoren bilden, die sich um eine Polrichtung (D1) der Kapsel (1A; 10; 20; 30) abwechseln.

12. Reihe von Kapseln (1A; 10; 20; 30) nach Anspruch 11, bei der sich die Sektoren im Wesentlichen zwischen einem Pol (N) der Kapsel (20; 30) und einem gegenüberliegenden Pol (S) entlang der Polrichtung (D1) erstrecken, wobei die Sektoren im Wesentlichen begrenzt sind:
- entweder durch Meridiane der Kapsel (20; 30),
- oder durch gekrümmte Linien (24), die sich im Wesentlichen zwischen dem Pol (N) und dem entgegengesetzten Pol (S) erstrecken.

13. Reihe von Kapseln (1A; 10; 20; 30) nach einem beliebigen der Ansprüche 10 bis 12, bei der die beiden äußeren Phasen (5A, 5B) der gelierten Hülle (5) mit dem Kern (3) in Kontakt sind.

14. Zusammensetzung, insbesondere kosmetische Zusammensetzung, eine Reihe von Kapseln (1A; 10; 20; 30) nach einem der Ansprüche 10 bis 13 und ein Medium zur Aufnahme dieser Kapseln (20; 30) umfassend.

15. Nicht-therapeutisches Verfahren zur kosmetischen Behandlung eines keratinischen Materials, insbesondere der Haut, mindestens einen Schritt des Auftragens mindestens einer Zusammensetzung nach Anspruch 14 auf das keratinische Material umfassend.

## Claims

1. Method for producing a series of capsules (1A; 10; 20; 30), wherein each capsule (1A; 10; 20; 30) comprises a core (3) which is liquid or at least partially gelled or at least partially thixotropic, and a gelled envelope (5) completely encapsulating the core (3) and comprising at least two external phases (5A, 5B), wherein each external phase (5A, 5B) contains at least one gelled polyelectrolyte, and wherein the gelled envelope (5) has an external surface (7) having at least two distinct portions (7A, 7B) respectively formed by the two external phases (5A, 5B), wherein the method comprises the steps of:
- conveying a first flow (F1) of one or more internal phases (3A, 3B) that are intended to form the core (3), and a second flow (F2) of at least the two phases (5A, 5B) that are intended to form the gelled envelope (5), wherein each external phase contains the liquid polyelectrolyte capable of gelling, at least one of the two external phases (5A, 5B) comprising at least one coloring agent, wherein the second flow (F2) surrounds the first flow about an axis (D2);
- successive formation, from the first flow (F1) and the second flow (F2), of a plurality of liquid bodies (57), wherein each liquid body (57) comprises a drop (107) of the internal phase(s) (3A, 3B), and a peripheral film (109) coating the drop and having the two external phases (5A, 5B), wherein the film (109) has on its external surface at least two distinct portions (109A, 109B) formed respectively by the two external phases (5A, 5B); and
- immersing each liquid body (57) in a gelling solution (100) containing a reagent adapted to react with the polyelectrolyte of each of the two external phases (5A, 5B) to form one of the capsules (1A; 10; 20; 30), and recovering the plurality of capsules (1A; 10; 20; 30).

2. Method according to claim 1, further comprising applying a gaseous flow (63) to each of the liquid bodies (57) being formed in order to deform the two portions (109A, 109B) of the external surface of the film (109) by rotation about the axis (D2), wherein the gaseous flow has a tangential component (T) with respect to the axis (D2).

3. Method according to claim 1 or 2, wherein during the step of forming the liquid bodies (57), the two external phases (5A, 5B) are in contact with the first flow (F1).

4. Method according to any one of the claims 1 to 3, wherein:
- the conveying is carried out in at least one nozzle (55) defining the axis (D2), wherein each of the two external phases (5A, 5B) is in contact with an external envelope (77) of the nozzle (55) at the outlet of the nozzle (55), and
- the liquid bodies (57) are formed at an outlet of the nozzle (55), wherein the method comprises a step of detaching the liquid bodies (57) from the nozzle (55).

5. Method according to claim 4, wherein the conveying step comprises:
- letting out the second flow (F2) from at least one collector (95) formed by the nozzle (55), wherein the collector (95) surrounds the first flow (F1) about a local circulation direction (D3) of the first flow (F1), and
- feeding the collector (95) from at least two sources (45A, 45B) supplying the two external phases (5A, 5B) to create a plurality of distinct supply areas (105A, 105B) in the collector (95), wherein each of the supply areas (105A, 105B) is filled by one or the other of the two external phases (5A, 5B), the supply areas (105A, 105B) filled by one alternating with those filled by the other around the local circulation direction (D3).

6. Method according to claim 5, wherein the nozzle (55) forms a plurality of supply channels (91, 93) of the collector (95), and wherein each supply channel (91, 93) is connected to the one of the two sources (45A, 45B) to form an inlet port in the collector (95), wherein the inlet ports are angularly successive about the local circulation direction (D3).

7. Method according to any one of the claims 5 or 6, wherein one of the supply areas (105A, 105B) is supplied at a first flow rate in one or other of the two external phases (5A , 5B), and another of the supply areas (105A, 105B) is supplied at a second flow rate in one or other of the two external phases (5A, 5B), wherein the first flow rate is lower than the second flow rate.

8. Method according to claim 7, wherein the ratio of the first flow rate divided by the total flow rate of the second flow (F2) is less than 1/3, preferably less than 1/4, or even less than 1/6, especially less than 1/8, and more preferably less than 1/10.

9. Method according to any one of the claims 4 to 8, wherein the conveying step comprises a physical separation of the first flow (F1) and the second flow (F2) at the outlet of the nozzle (55) by an envelope (301, 401) of the nozzle (55).

10. Series of capsules (1A; 10; 20; 30) capable of being manufactured by a method according to any one of the claims 1 to 9, wherein each capsule (1A; 10; 20; 30) comprises:
- a core (3) which is liquid or at least partially gelled or at least partly thixotropic, wherein the core (3) comprises one or more internal phases (3A, 3B), and
- a gelled envelope (5) completely encapsulating the core (3) and comprising at least two external phases (5A, 5B), wherein each external phase contains a gelled polyelectrolyte, wherein at least one of the two external phases (5A, 5B) comprises a coloring agent, wherein the gelled envelope (5) has an external surface with at least two distinct portions (7A, 7B) respectively formed by the two external phases (5A, 5B).

11. Series of capsules (1A; 10; 20; 30) according to claim 10, wherein, for each capsule (1A; 10; 20; 30), the external surface (7) of the gelled envelope (5) comprises a plurality of portions (7A, 7B) formed by one or the other of the two external phases (5A, 5B), wherein the portions (7A, 7B) form sectors alternating about a polar direction (D1) of the capsule (1A; 10; 20; 30).

12. Series of capsules (1A; 10; 20; 30) according to claim 11, wherein the sectors extend substantially between a pole (N) of the capsule (20; 30) and an opposite pole (S) according to the polar direction (D1), wherein the sectors are approximately delimited:
- either by meridians of the capsule (20; 30),
- or by curved lines (24) extending substantially between the pole (N) and the opposite pole (S).

13. Series of capsules (1A; 10; 20; 30) according to any one of claims 10 to 12, wherein the two external phases (5A, 5B) of the gelled envelope (5) are in contact with the core (3).

14. Composition, in particular a cosmetic composition, comprising a series of capsules (1A; 10; 20; 30) according to any one of claims 10 to 13, and a medium for receiving these capsules (20; 30).

15. Non-therapeutic method for cosmetic treatment of a keratinous material, in particular the skin, comprising at least one step of applying to the keratinous material at least one composition according to claim 14.
